# EUROPEAN PATENT SPECIFICATION

(11) **EP 0 154 344 B1**
(45) Date of publication and mention of the grant of the patent: **07.07.1993**
(21) Application number: 85102607.0
(22) Date of filing: 07.03.1985
(51) Int. Cl.: A61K 31/165, A61K 31/195

(54) **Antiviral pharmaceutical preparations and their use**
Antivirale pharmazeutische Zusammensetzungen und ihrer Verwendung
Compositions pharmaceutiques antivirales et leur utilisation

(30) Priority: 08.03.1984 US 587398
(43) Date of publication of application: 11.09.1985
(73) Proprietor: Brigham, Dana, North Bay Village Miami Florida 33141 (US)
(72) Inventor: Haines, Harold Gray, Miami Florida 33176 (US); Dickens, Caroline Burgess, Miami Florida 33156 (US)
(74) Representative: Werner, Hans-Karsten, Dr.Dipl.-Chem.

(56) References cited:
- FR-A- 2 484 257
- GB-A- 983 558
- Egypt. J. Pharma. Sci., 1980, 18(4), p.355-66
- Pediatria, 1965, 8(2), p. 147-58
- Ther. Ggw., 1970, 109(2), p. 248-58
- Drug. Dev. Ind. Pharm., July 1984, 10(4), p. 685-97
- Microbiol. Immunol., vol. 28, no. 2, Feb. 1984, p. 243-249, T. Uchida et al.
- J. Dermatol., vol. 7, no.2, Apr. 1980, p. 161-162, Tokyo, JP, A. Ogata et al.
- Br. Med. J., vol. 1, no. 6166, March 1979, p. 818, M. Schreuder et al.
- Curr. Ther. (Australia), vol. 18, no 3, 1977, p. 61-65, W.A. McIndoe.
- Pediatr. Clin. North Am., vol. 25, no.2, May 1978, p. 339-355, M. Jarratt et al.
- J. Am. Acad. Dermatol., vol. 8, no.3, March 1983, p. 433-436, B.H. Thiers.
- Obstet. Gynecol., vol. 36, no.4, Oct. 1970, p. 645-651, B.p. Alan et al.
- Calif. Med., vol. 103, no.4, Oct. 1965, p. 277-279., M.J. Marmer.
- Chemical Abstracts, vol. 99, 1983, p. 341, abstract 110754a, Columbus, Ohio, US & RO-A-75 309 (Intreprinderea de Antibiotice) 25-03-1982.

## Description

This invention relates to the use and a pharmaceutical composition for inhibiting herpes viral infection in mammals, with specific emphasis upon the treatment of herpes simplex virus infections in humans. Four separate herpes group viruses, which infect and cause disease in humans can thus be treated: these are (1) herpes simplex virus 1 and 2; (HSV-1 and HSV-2); (2) cytomegalovirus (CMV); (3) varicella-zoster virus (VZ); and (4) and Epstein-Barr virus (EB).

We have discovered that an amino-amide, which is commonly called lidocaine (2-diethylamino-2',6'-acetoxylidide), is an effective antiviral agent in cell culture against HSV-1 and HSV-2 and is able to treat herpes virus infections of mammals. It is particularly effective in the treatment of HSV oral and genital lesions of humans.

Furthermore, we have discovered that the addition of pantothenic acid or its alcohol and salt forms, dexpanthenol and pantothenate respectively, to lidocaine or lidocaine hydrochloride significantly enhances the antiviral activity of those drugs.

Ng et al. (Obstetrics and Gynecology, V. 36, pp. 645-651, 1970) used 1% lidocaine hydrochloride for symptomatic treatment of genital herpes virus infection in women. They reported no beneficial results due to this therapy. The failure of Ng et al. to observe beneficial effects from topical lidocaine HCl therapy is unexplained but may have been due to lack of adequate concentrations of the compound.

Vucina et al. (Pediatria, Vol 8 (2), pp. 147-50, 1965) describe the use of panthenol in the treatment of nine cases of herpes zoster and concluded that panthenol had an important analgesic effect, that no new lesions occurred during the course of treatment, and that the duration of the clinical symptoms was less than that observed with other types of treatments. In these clinical trials, they used either 5% topical application or 5% injectable solution. They did not report on the usage of panthenol in the treatment of herpes simplex virus infection.

Microbiol. Immunol., Vol. 28, No. 2, February 1984, pages 243 - 249 describes a study which examined the inhibitory effects of aliphatic amines on the syncytium formation induced by HSV-1 and compared their effects with those of local anesthetics, tranquilizers and ammonium salts. Under several compounds as local anesthetic lidocaine is used. The reference is directed to inhibition of cell fusion and suggests that local anesthetics are inhibitory in the formation of herpetic syncytia.

J. Dermatol. (Tokyo), Vol. 7, No. 2, April 1980, pages 161 - 162 describes the use of lidocaine as local anesthetica for patients who suffer from intolerable pain.

In J.Am. Acad. Dermatol., Vol. 8, No. 3, March 1983, pages 433 - 436 a study of colding is cited which proposes to use lidocaine with noradrenaline to perform sympathetic nerve block in patients with acute herpes zoster.

Calif. Med., Vol. 103, No. 4, October 1965, pages 277 - 279 describes a treatment of acute herpes zoster by continuous epidural analgesia.

Egypt. J. Pharm. Sci., Vol. 18, No. 4, pages 355 - 514, Cairo 1980 describes a method for the determination of lidocaine in pharmaceutical preparations with a help of bromocresol green at optimum pH of 4.2. Furthermore the interference of the bromocresol green assay was studied resulting from pantenol and other substances.

Pediatria, 1965, 8 (2), pages 147 - 158 discloses the use of pantenol in the treatment of herpes zoster. It is reported that the pain occuring with a herpes zoster infection was reduced and that there was a duration of the clinical course of the disease.

The present invention according to claim 1 concerns the use of lidocaine or a pharmaceutically acceptable salt thereof for the preparation of a pharmaceutical composition for the inhibition of herpes viral infections in mammals. Good results are achieved by administering to the mammal an effective (preferably 1.5 - 10% w/v with respect to the pharmaceutical composition) herpes antiviral amount of lidocaine. Better results are achieved by administering, either in topical or parenteral form, an effective antiviral amount of lidocaine or a pharmaceutically acceptable salt thereof in combination with panthenol, pantothenic acid or its salt forms.

The preparations comprise a combination of an antiviral amount of lidocaine or a pharmaceutically acceptable salt thereof alone or in combination with panthenol, pantothenic acid or a pharmaceutically acceptable salt thereof, e.g. with metals such as sodium, potassium, calcium, or aluminum or with bases such as tri-(C₁₋₆-alkyl)amines (e.g. triethylamine), ammonium, guanidine.

### DETAILED DESCRIPTION OF THE INVENTION

Lidocaine was the first amino-amide to demonstrate clinical usefulness as a local anesthetic and many topical anesthetics that have been synthesized since the introduction of lidocaine are also amino-amides. The following compounds which are chemically related to lidocaine have been introduced into clinical practice in recent years: prilocaine (2-(propylamino)-2'-propionotoluidide); etidocaine (2-ethylpropylamino-2',6'-butyroxylidide); mepivacaine (1-methyl-2',6'-pipecoloxylidide) and its homolog bupivacaine (1-butyl-2',6'-dimethyl-2-piperidinecarboxanilide).

Lidocaine and its pharmaceutically active salts have been found by the present inventors to exhibit antiviral activity in cell culture and animal model systems at a concentration ranging from 0.5 mg/ml (.05%) to 100 mg/ml (10%). The recommended intramuscular daily dosage in humans is 4.3 mg/kg of body weight or 2.0 mg per pound of body weight. Thus, in a 70 kg man, the recommended daily dosage is 300 mg. This generally is injected in a single intramuscular bolus form. In topical (ointment or solution) form, a representative dosage would be the application of a 1.5 to 10% topical application (most desirably 1.5 to 4%) 3 to 4 times daily. When pantothenic acid or dexpanthenol is included in the formulation, it is preferably present at a concentration of from 5-50 mg/ml.

When lidocaine is administered in the form of a pharmaceutically acceptable salt, the salt will be a non-toxic salt, suitably an acid addition salt of an inorganic acid or strong organic acid, e.g. a hydrohalide, sulfate, nitrate, phosphate, acetate, tartrate, citrate, fumarate, maleate, toluenesulfonate or methanesulfonate.

While it is possible for lidocaine or its pharmaceutically acceptable salt to be administered as the raw material, it is preferably administered in the form of a pharmaceutical formulation. The pharmaceutical formulation will comprise the active compound, together with a pharmaceutically acceptable carrier. The carrier must be "acceptable" in the sense of being compatible with the other ingredients of the formulation and not deleterious to the recipient thereof. The pharmaceutical formulations may be prepared by any of the methods well known in the art of pharmacy.

When the pharmaceutical formulation is applied topically, the carrier may suitably comprise a solution, ointment, or gel base. The base, for example, may comprise one or more the following: petrolatum, lanolin, polyethylene glycols, bees wax, mineral oil, diluents such as water and alcohol, and emulsifiers and stabilizers. Topical formulations may contain a concentration of the lidocaine or lidocaine salt from 1.5 to 10% w/v (by weight per unit volume). Pharmaceutical formulations suitable for parenteral administration include sterile solutions or suspensions of the active compound in water or other suitable vehicles.

### IDENTIFICATION OF LIDOCAINE AS AN ANTIVIRAL SUBSTANCE

### A. CELL CULTURE

The following experiments were specifically designed to determine if lidocaine hydrochloride possesses antiviral activity against the replication of herpes simplex virus Type 1 in Vero cell cultures. Harvest experiments were done in which HSV-1 infected cell cultures were post-treated with lidocaine hydrochloride at a number of concentrations. The vessels containing the cells were harvested when the cytopathic effect (CPE) in the untreated virus (negative) control cultures was 3+ to 4+ (approximately 90% of cells showing CPE). The positive control in these experiments was 5-iodo-2'-deoxyuridine (IUDR). The viral harvests were then plaque assayed on vero cells to determine the number of plaque forming units per milliliter (pfu/ml) produced in each test flask as compared to the controls. Table 1 shows the results of these harvest titrations and clearly points out that lidocaine hydrochloride is able to prevent the replication of herpes simplex virus Type 1 in cell culture, therefore establishing it as an antiviral agent. Table 1 gives the results of various concentrations of lidocaine hydrochloride from 0.4 to 1.8 mg/ml. Significant antiviral activity is seen throughout this range of concentrations. At a concentration of 2.0 mg/ml, lidocaine hydrochloride was toxic in cell cultures. Using decreasing concentrations of lidocaine hydrochloride, antiviral activity of lidocaine hydrochloride was found to be significant down to 0.1 mg/ml.

**TABLE 1**

| DOSE-RESPONSE OF LIDOCAINE HCl VERSUS HSV-1 (MOI-1.0) | | | | |
|---|---|---|---|---|
| COMPONENT | *CONCENTRATION | HARVEST | | |
| | | PFU/ML | LOG VIRUS | LOG REDUCTION |
| Virus Control | ---- | 1.4 x 10⁶ | 6.2 | --- |
| Idoxuridine | 10 ug/ml | 7.5 x 10³ | 3.9 | 2.3 |
| Lidocaine HCl | 2.0 mg/ml | Toxic | --- | --- |
| Lidocaine HCl | 1.8 mg/ml | 0 | 0 | 3.0 |
| Lidocaine HCl | 1.6 mg/ml | 0 | 0 | 3.0 |
| Lidocaine HCl | 1.4 mg/ml | 0 | 0 | 3.0 |
| Lidocaine HCl | 1.2 mg/ml | 0 | 0 | 3.0 |
| Lidocaine HCl | 1.0 mg/ml | 0 | 0 | 3.0 |
| Lidocaine HCl | *0.8 mg/ml | 4.5 x 10³ | 3.7 | 2.5 |
| Lidocaine HCl | 0.4 mg/ml | 3.7 x 10⁴ | 4.6 | 1.6 |

Because the herpes simplex virus replication cycle occurs in a well-described, orderly temporal fashion, one approach to estimating the stage of replication blocked by lidocaine hydrochloride is to perform a series of post-infection treatments with the agent, in a one-step replication cycle. In this experiment, viral cell cultures were infected with HSV-1 at a multiplicity of 3 and allowed to adsorb for one hour. The inoculum was then removed, and duplicate infected cultures were then treated at 1, 2, 4, 6, 8, 10, 12, 14 and 16 hours post-infection with 1.8 mg/ml of lidocaine hydrochloride in maintenance medium. At 18 hours, all cultures were washed three times with phosphate buffered saline. One milliliter of phosphate buffered saline was added back to the monolayer, and the cultures were then harvested by three cycles of freeze-thawing followed by centrifugation. The viral harvest was then titrated on viral cells, and the pfu/ml of harvest virus was calculated. The control in this experiment was a one-step multiplication cycle of untreated viral cells also infected but harvested at the same time as the treated cultures. It was found that treatment of HSV-1 infected cells with 1.8 mg/ml of lidocaine as late as 12 hours after infection is able to cause significant reduction (48%) in virus replication. When treated up to 8 hours post-infection, over 95% of virus production was inhibited. Even at 10 hours post-infection, 84% of virus production was inhibited. Because of this unusual effect, it is possible that lidocaine may act somewhat late in the herpes replication cycle unlike many effective herpes simplex antiviral drugs such as the nucleotide analogs previously described.

When dexpanthenol, in concentrations ranging from 0.6 mg/ml to 9.6 mg/ml, was tested against herpes simplex virus Type 1 in cell culture using the same assay system as described for lidocaine hydrochloride, a marked antiviral activity was seen for concentrations of dexpanthenol above 1.2 mg/ml. It is significant that 9.6 mg/ml of dexpanthenol showed a reduction in viral titer of greater than 99.99% of the original amount of virus. Furthermore, in separate experiments, when 0.4 mg/ml of lidocaine was mixed with 0.8 mg/ml of dexpanthenol, a reduction in virus titer 50% greater than that seen with lidocaine or dexpanthenol at the same concentrations alone was observed.

### NEW ANTIVIRAL EFFECT TESTS MATERIAL AND METHODS

### CELLS

African green monkey kidney calls (Vero cells) were grown in Eagle's minimal essential medium supplemented with 2 mM L-glutamine, 10 ug/ml gentamycin, 0.25 mg/ml amphotericin B (Fungisone) and 10% heat-inactivated fetal calf serum. Cells were grown at 37°C in a humidified 5% CO₂ atmosphere.

### VIRUSES

Herpes simplex virus Type 1 (HSV-1), Strain Dorr from our stocks was used.

Stocks of viruses were grown by infecting Vero cells at a multiplicity of 0.01 pfu/cell, and harvested in media as above except that 2% fetal calf serum was used. Virus was harvested by three cycles of freezing and thawing, followed by centrifugation at 3000 rpm at 4°C for 15 minutes to remove cell debris. The supernatants were aliquoted and frozen at -70°C.

HSV-1 and 2 were assayed by serial dilutions in PBS followed by adsorption of 0.1 ml of virus onto confluent monolayers of Vero cells in 35 mm² plates previously washed three times with PBS. Following a 60 minute adsorption period at 37°C, the virus inoculum was removed, the infected monolayers were washed twice with PBS, and covered with 2 mls of 1.5% pooled human sera in MEM maintenance media. At 72 hours post-infection, the overlay was removed, and the monolayers washed three times with PBS. The cells were stained with 0.2% crystal violet solution containing 95% methanol and 1% ammonium oxalate in water, rinsed with tap water, dried, and the plaques were counted.

### ANTIBODIES

Rabbit-anti-HSV-1 (Strain MacIntyre) antibody and goat-anti-rabbit fluorescein isothiocyanate conjugate antibody were obtained commercially.

### EFFECT OF LIDOCAINE ON CELL ATTACHMENT

Cells were seeded at a density of 5 x 10² or 5 x 10¹ cells in 4 mls of lidocaine-containing growth media into 60 mm dishes. The plates were incubated at 37°C for 7 days to allow colonies to form. The media was then removed, the cells were washed three times in Ca⁺⁺ and Mg⁺⁺ free phosphate buffered saline (PBS), stained with crystal violet, rinsed, and dried. The colonies were counted using a stereoscopic microscope. The minimum number of cells defined as a colony was 50, and the average colony contained 100 cells. The mean % survival was calculated by dividing the mean number of colonies in treated cultures by the mean of colonies from untreated control cultures.

### INDIRECT IMMUNOFLUORESCENCE

Four systems were analyzed:
a) Uninfected untreated Vero cells
b) Uninfected lidocaine-treated (1.0 mg/ml) Vero cells
c) HSV-1 infected untreated Vero cells (MOI = 0.3)
d) HSV-1 infected treated Vero cells (1.0 mg/ml lidocaine MOI = 0.3)
Cultures were treated at 1 hour post-infection. At 18 hours post-infection, the cultures were fixed with cold methanol, stained with rabbit anti-HSV-1 antibody for 30 minutes, washed three times with PBS, stained for 30 minutes with fluorescein conjugated goat anti-rabbit antibody, washed, and observed with the fluorescent microscope.

### LIVE CELL SURFACE INDIRECT IMMUNOFLUORESCENCE ANALYSIS

Four systems were analyzed:
1) Uninfected, untreated Vero cells
2) Uninfected lidocaine-treated Vero cells
3) HSV-1 infected, untreated Vero cells
4) HSV-1 infected, lidocaine-treated Vero cells
Cells were grown in 25 cm² flasks to confluency. The growth media was removed, cells washed three times in PBS, and infected with HSV-1 at an MOI of 3.0 or mock-infected. After a 60 minute adsorption period, the inoculum was removed, the cells washed once in PBS, and lidocaine-containing or control maintenance media was added. The concentration of lidocaine in treated cultures was 1.0 mg/ml. The cells were incubated at 37°C for 18 hours. The media was then removed, the cells washed once with PBS, then treated with 5 mls of 0.02% EDTA in PBS and shaken to disperse the monolayers.

Cells were resuspended in the flasks, then transferred to 15 ml centrifuge tubes and spun at 800 rpm for 5 minutes at room temperature to pellet the cells. The EDTA supernatant was removed, and two PBS washes followed by the same centrifugation method. The cell pellets were resuspended in 5 mls of PBS and divided into two suspensions of 2.5 mls for staining.
Two staining protocols were then carried out:
1) Goat-anti-rabbit FITC antibody only
2) Rabbit anti-HSV and goat anti-rabbit FITC antibodies.

The 2.5 ml cell suspensions were again centrifuged as above, and the cell pellets resuspended in 1.0 ml of:
1) Goat-anti-rabbit FITC conjugate, or
2) Rabbit anti-HSV antibody.

Both the rabbit anti-HSV-1 antibody and the goat-anti-rabbit FITC antibody were used at a final dilution of 1:40. This dilution was determined in a prior experiment in which a checkerboard titration of the antibodies utilizing cross-dilutions of 1:20 through 1:160 were used to stain uninfected and HSV-1 infected cells at an MOI of 0.3. Also included were uninfected and HSV-infected cultures stained with the goat-anti-rabbit FITC antibody only. The optimal concentration of antibodies was considered to be dilutions which produced the least non-specific (background) staining and the best visualization of cell structures by fluorescent microscopy.

The cultures were held at room temperature in the dark for 30 minutes to allow antibody binding, then centrifuged at 500 rpm for 5 minutes. The cells were then washed three times with 5 mls of PBS as above. For staining protocol, the staining procedure was then repeated as above with the goat-anti-rabbit FITC antibody. The final cell pellets were resuspended in 1 ml of PBS and divided into two 0.5 ml of 10% formalin in PBS. All samples were then observed by fluorescent microscopy, then subjected to automated fluorescence analysis. The observed samples were quantitated by counting 20 cells per sample and graded as to relative observed fluorescence on a scale of 0 to 6, and the mean relative fluorescence was calculated for each sample.

### AUTOMATED FLUORESCENCE ANALYSIS

Prior to automated fluorescence analysis, it was determined by fluorescent microscopy that the formalin fixed samples were equivalent in fluorescent intensity and number of fluorescing cells to the unfixed samples and that the cell morphologies were unchanged by the fixing process. Therefore, following confirmation of the fluorescence parameters of the two groups in an automated analysis, the formalin fixed samples were selected for the final automated fluorescence analyses.

Eight samples were analyzed using the following code:

### Antibodies

1) Goat-anti-rabbit FITC antibody (2nd antibody only)
2) Rabbit-anti-HSV-1 and goat-anti-rabbit FITC antibody (1st and 2nd antibodies)

### Samples

- UU-1: - Uninfected, untreated cells, 2nd antibody only
- UT-1: - Uninfected, lidocaine-treated cells, 2nd antibody only
- UU-2: - Uninfected, untreated cells, 1st and 2nd antibodies
- IU -1: - HSV-1 infected, untreated cells, 2nd antibody only
- IT-1: - HSV-1 infected, lidocaine-treated cells, 2nd antibody only
- IU-2: - HSV-1 infected, untreated cells, 1st and 2nd antibodies
- IT-2: - HSV-1 infected, lidocaine-treated cells, 1st and 2nd antibodies

The UU-1, UU-2 controls were designed to determine whether either antibody system bound to uninfected, untreated Vero cells. The UT-1, UT-2 controls were designed to determine if lidocaine was fluorescent in itself or caused fluorescence in the presence of the antibody systems. The IU-1, IU-2 controls were designed to confirm the specificity of the antibodies as selected. The IT-1 control was designed to determine if HSV-infected lidocaine treated cells bound the 2nd antibody significantly, and the IT-2 sample was designed to determine if lidocaine affected the amount of HSV-specific antibody binding as compared to the IU-2 sample.

The analyzer parameters were as follows:

| | |
|---|---|
| Cells counted: | 10,001 |
| Laser Power: | 500 W at 488 nm's |
| Nozzle Size: | 50 |

The criteria for positive fluorescence was considered to be the area of positives where there was a less than 5% overlap in the negative controls. The analysis was designed to measure linear fluorescence as a function of the relative cell number of the population examined at a specific channel. The mean peak fluorescence channel was calculated as the mean fluorescent intensity for the population examined. The number of positive cells was calculated by multiplying the percent positive cells in a given area by the total number of cells analyzed. The results were tested using Kolmogorov-Smirnoff statistics and the null hypothesis at the 99.9% confidence level, corresponding to a p (or alpha) value less than .001.

### DIRECT INACTIVATION OF HSV-1 by LIDOCAINE

Three concentrations of lidocaine were incubated with 9 x 10⁶ plaque-forming units of HSV-1 in MEM at 4°C for 1 hour. Appropriate dilutions were made, and the virus titrated by plaque assay.

### INFECTIOUS CENTER ASSAY

Cells were washed three times in PBS and infected with HSV-1 at an MOI of 3.0 in 25 cm² flasks. After a 60 minute adsorption period, the inoculum was removed, the cells washed, and 1.5, 1.0, 0.5, or 0.0 mg/ml lidocaine in maintenance media was added. Following an 18 hour incubation period at 37°C, the cultures were washed and dispersed with trypsin-EDTA. Dilutions of the resuspended cells were made and mixed with uninfected Vero cells and plated using maintenance media containing 1.5% pooled human sera (HSV overlay). The overlay was then removed, the cells washed in PBS and stained with crystal violet. The number of infective centers was determined using a stereoscopic microscope.

### ONE-STEP REPLICATION CYCLE

Confluent Vero cell cultures were washed in PBS and infected with HSV-1 at an MOI of 3.0 in 35 mm² dishes. Immediately following virus inoculation, three cultures were washed and prepared for harvesting intracellular virus by washing with PBS twice, adding 1.0 ml of PBS to each culture and freezing at -70°C.

Three series were examined:
1) Lidocaine treated cultures (1.0 mg/ml concentration)
2) Iododeoxyuridine treated cultures (10 ug/ml concentration)
3) Untreated cultures
Following a 60-minute adsorption period, the inoculum was removed, the cells washed and treated with the appropriate compound in maintenance medium or maintenance media only (control cultures). Three cultures of each series were then prepared for harvesting intracellular virus (one hour harvest). At 2, 4, 6, 8, 10, 12, 14, 16, and 18 hours post-infection, three cultures of each series were prepared for harvesting. All cultures were then harvested by three cycles of freeze-thawing with scraping of the cells from the dishes followed by centrifugation at 3000 rpm for 30 minutes at 4°C to pellet cell debris. The supernatants were stored at -70°C, and the amount of intracellular virus produced at each time point was determined by plaque assay.

### EFFECT OF TIME OF TREATMENT ON REPLICATION

Confluent Vero cell cultures in 35 mm² plates were washed in PBS and infected with HSV-1 at an MOI of 3.0.
Two series were analyzed:
1) Lidocaine treated cultures (1.0 mg/ml concentration)
2) Iododeoxyuridine treated cultures (10 ug/ml concentration)
After a 60 minute adsorption period, the inoculum was removed, the cells washed in PBS, and three cultures for each series were treated with the appropriate drug in maintenance media. The remaining cultures were overlaid with maintenance medium. At 2, 4, 6, 8, 10, 12, 14, 16, and 18 hours post-infection, the media was removed from three cultures of each series, the cells were washed in PBS, and the media replaced with either the lidocaine or iododeoxyuridine-containing media. All cultures were incubated at 37°C for 18 hours. The 18 hour post-treatment plates were exposed to either drug for approximately two minutes. At 18 hours post-infection, the media was removed and all cultures harvested for intracellular virus. Six HSV-1 infected, untreated cultures were also harvested at 18 hours. The effect of the time of treatment of both drugs on HSV-1 replication was determined by plaque assay.

### RESULTS

### TOXICITY OF LIDOCAINE TO UNINFECTED CELLS

Lidocaine did not inhibit the colony forming ability of Vero cells at concentrations of 0.05 mg/ml or lower. Treatment of Vero cells with lidocaine at concentrations of 2.0 mg/ml or above reduced the colony forming ability by greater than 90%. Concentrations of lidocaine of 1.0 mg/ml or less produced mean survival values of 75% or greater. The 50% inhibitory dose was calculated from generation of a linear regression plot and was found to be 1.28 mg/ml.

Because of these results, and because concentrations of 1.0 mg/ml or less of lidocaine significantly inhibit the replication of herpes simplex in Vero cells, a concentration range of 1.0 mg/ml ( 3 log reduction) to 0.05 mg/ml (50% plaque reduction) was selected for use in the antiviral assays using other viruses.

### INHIBITION OF HSV-1 REPLICATION BY LIDOCAINE

Vero cells were infected with HSV-1 Strain Dorr at an MOI of 3. At 1 hour post infection, maintenance media containing lidocaine was added and the cultures incubated for 24 hours. The virus was harvested, and the virus yield was determined by plaque assay on Vero cells.

Treatment of HSV infected Vero cell cultures with concentrations of lidocaine of 0.05 mg/ml inhibited the plaque formation units per milliliter (pfu/ml) by 50%. This dose of lidocaine was noted to be noncytotoxic. A reduction of viral harvest of 2 logs was observed for 0.6 mg/ml, a concentration which was 11% cytotoxic. Concentrations of 1.0 mg/ml lidocaine produced viral harvest titers of less than 5 x 10² plaque forming units per ml, and this concentration corresponds to a cytotoxicity of only 26%.

### INHIBITION OF HSV-2 REPLICATION BY LIDOCAINE

Vero cells were infected with HSV-2 (clinical isolate) at an MOI of 3. At 1 hour post-infection, maintenance media containing lidocaine were added, and the cultures were incubated for 24 hours. The virus was harvested, and the virus yield was determined by plaque assay on Vero cells.

Treatment of HSV-2 infected cultures with concentrations of lidocaine from 0.05 mg/ml to 1.0 mg/ml produced increasing reductions in viral harvest titers from 0.67 logs to 2.6 logs, respectively.

### EFFECT OF LIDOCAINE ON CELL ATTACHMENT

Vero cells were plated at a density of 5 x 10² cells in 4 mls of lidocaine-containing growth media into 60 mm dishes. After 7 days of incubation at 37°C, the media was removed, the colonies washed three times with PBS, and stained with crystal violet. The mean of two experimental determination is plotted.

Lidocaine did not significantly inhibit Vero cell attachment and subsequent colony formation at concentrations of 1.0 mg/ml or lower. However, at concentrations of 1.2 mg/ml and above, the colony forming ability of cells plated in the presence of lidocaine was increasingly inhibited.

The 50% inhibitory dose generated from a linear regression analysis was 1.28 mg/ml. The 50% inhibitory dose generated from a linear regression analysis of a cell attachment plot was 1.02 mg/ml. However, because these experiments determined that the presence of drug at some concentrations at the time of plating and for 5 additional days of treatment reduced colony formation, the values cannot be directly compared to determine the difference between results of attachment versus those of cytotoxicity.

### INDIRECT IMMUNOFLUORESCENCE ANALYSIS

Four separate determinations were made at concentrations from 0.2 through 1.0 mg/ml lidocaine. Three observers examining the coded slides concluded independently that the fluorescence at all concentrations of lidocaine appeared to be similar in the number of fluorescing cells and in their intensity to the positive controls. It was concluded that HSV-1 antigens appeared to be synthesized near untreated control levels within Vero cells.

### LIVE CELL SURFACE INDIRECT IMMUNOFLUORESCENCE OF LIDOCAINE-TREATED HSV-1 INFECTED VERO CELLS

Uninfected Vero cells did not significantly bind either a rabbit-anti-HSV antibody or a goat anti-rabbit antibody system utilized. Neither did lidocaine-treated, uninfected cells fluoresce, indicating that non-specific antibody binding and lidocaine-induced fluorescence did not occur.

Staining protocol 1 results showed that the conjugate antibody did not bind non-specifically to Vero cells, whether HSV-infected or uninfected. The results indicated that there was a decrease in fluorescent intensity on the surface of lidocaine-treated HSV-1 infected cells as compared to untreated, infected cells. It was concluded that lidocaine prevented HSV-specific antigen accumulation on the surface of live cells.

### LIVE CELL SURFACE INDIRECT IMMUNOFLUORESCENCE ANALYZED BY AUTOMATED FLUORESCENT FLOW CYTOMETRY

Uninfected cells stained with second antibody only or both antibodies did not show positive fluorescence. Uninfected, lidocaine-treated cells also did not fluoresce with either staining system. HSV-infected cells, whether treated or untreated with lidocaine, did not show positive fluorescence when stained with the second antibody only.

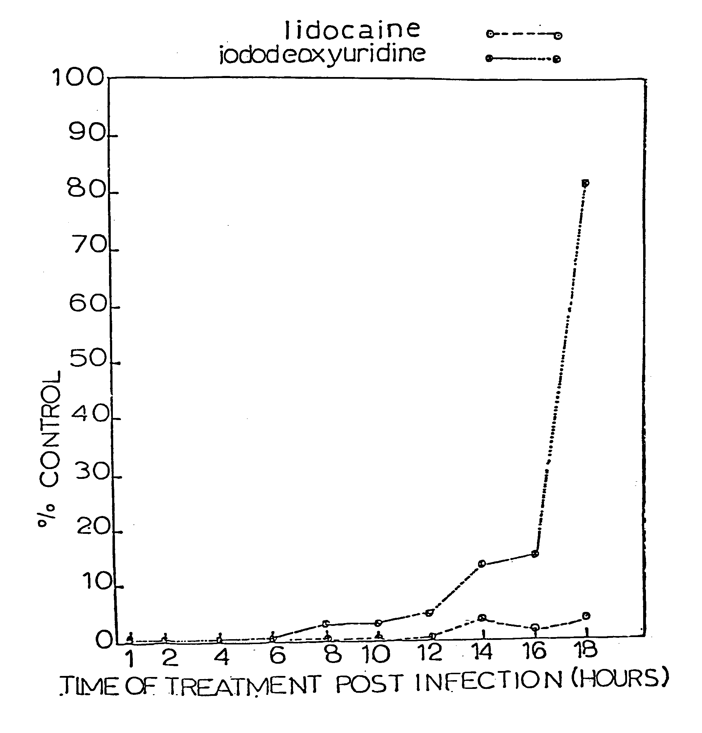

HSV-infected, untreated cells stained with both antibodies produced 90.8% positively staining cells (9,081 cells/10,001 total cells (Figure 1). The mean peak channel was 84.7. Thus, the linear fluorescence mean intensity for this population was approximately 85. The percent of cells above the mean was 45.08%, and the percent of cells below the mean was 45.73. HSV-1 infected, lidocaine-treated cells stained with both antibodies produced 47.67% positively staining cells (4,767 cells/10,001 total cells).

The mean peak channel was 62.2, and the mean linear fluorescence about 62. The percent of positive cells above the mean was 12.73%, and the percent of positive cells below the mean was 34.94%.

Channels 22 through 128 were integrated as these were determined to be fluorescence positive. In the lidocaine-treated sample, there was a 43% decrease in the number of positive cells, and a 22.5 mean channel shift, indicating a significant decrease in fluorescent intensity as well. These results indicate that lidocaine inhibits the insertion of HSV-specific antigens into plasma membranes.

### DIRECT INACTIVATION OF HSV-1 BY LIDOCAINE

Incubation of HSV-1 with the three doses of lidocaine at 4°C did not lower the viral titers (Table 2). It was concluded that lidocaine does not directly inactivate the infectivity of HSV-1 at 4°C.

### INFECTIOUS CENTER ASSAY OF HSV-1 INFECTED, LIDOCAINE-TREATED CELLS

Lidocaine inhibited the formation of infectious centers at all concentrations tested (Table 4). It was apparent from this experiment that HSV-infected cells post-treated with lidocaine, extensively washed, then co-cultivated with uninfected cells were unable to support active HSV replication except at very low levels (3 to 15% that of controls) for the equivalent of 4 replication cycles.

### ONE-STEP REPLICATION CYCLE

Intracellular HSV-1 titers for both the lidocaine and iododeoxyuridine-treated cultures were similar to those of the control cultures up through six hours post-infection. However, between 6 and 8 hours post-infection, a 1 log increase in titer occurred in the control cells which was followed by increasing titers to a final level of 1.15 x 10⁶ pfu/ml at 18 hours post-infection. The lidocaine harvests were initially lower than those of the IDU-treated harvests, and increased between 10 and 12 hours post-infection to levels above the IDU harvests, but the greatest difference noted was only 0.67 logs at 16 hours post-infection. It was concluded that both inhibitors significantly prevented intracellular virus replication increases normally beginning around 6 to 8 hours post-infection, as seen in the control harvests.

### EFFECT OF TIME OF TREATMENT ON REPLICATION

Iododeoxyuridine was effective in inhibiting HSV-1 replication to below 2% of control levels when given as late as 12 hours post-infection (p.i.). However, at 14 hours p.i., 13.7% harvest titers were measured, increasing to 81.3% of control values when cultures were treated at 18 hours p.i. (Figure 1).

Lidocaine was effective in inhibiting HSV-1 replication to 0.19% of control levels when given at 12 hours, p.i., and remarkably, produced viral harvest titers of between 1.2% and 3.1% of control levels at 14, 16, and 18 hours p.i. These results indicate that lidocaine either acts very late in the replication cycle or is independent of replication events.

### DISCUSSION AND RATIONALE

The data presented here indicated that lidocaine:
1) Is a potent inhibitor of intracellular HSV 1 and 2 and HSV replication.
2) Apparently does not inhibit intracellular HSV antigen accumulation to an appreciable degree.
3) Prevents the accumulation of HSV-specific antigens on the plasma membrane.
4) Exerts its antiviral action independent of the stage of viral replication occurring, or acts extremely late in the replication cycle.
5) Apparently does not directly inactivate virions, but
6) Is able to reduce viral yields to 3% of control levels after a 2 minute treatment of infected cells following one cycle of replication.

These results indicate that lidocaine may act by one of the following mechanisms:
1) Interferes with synthesis or processing of HSV-specific glycoproteins essential for infectivity.
2) Interferes with viral-induced transport of enveloped nucleocapsids to the rough endoplasmic reticulum and Golgi for glycosylation, or
3) Causes a biophysical alteration of viral specific glycoproteins, resulting in loss of infectivity.

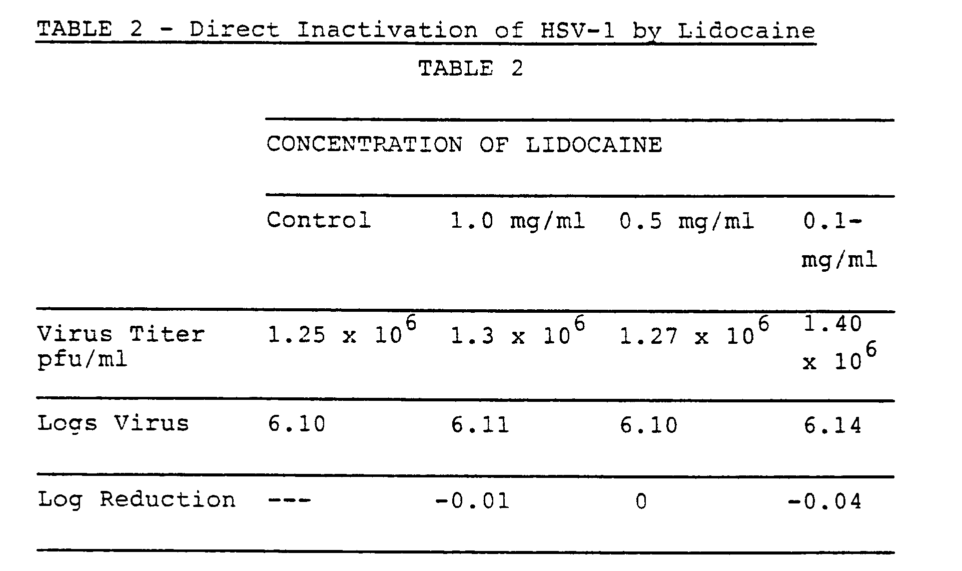

Three concentrations of lidocaine were incubated with 9 X 10⁶ plaque-forming units of HSV-1 at 4°C for 1 hour. Appropriate dilutions of the virus were then made and quantitated by plaque assay.

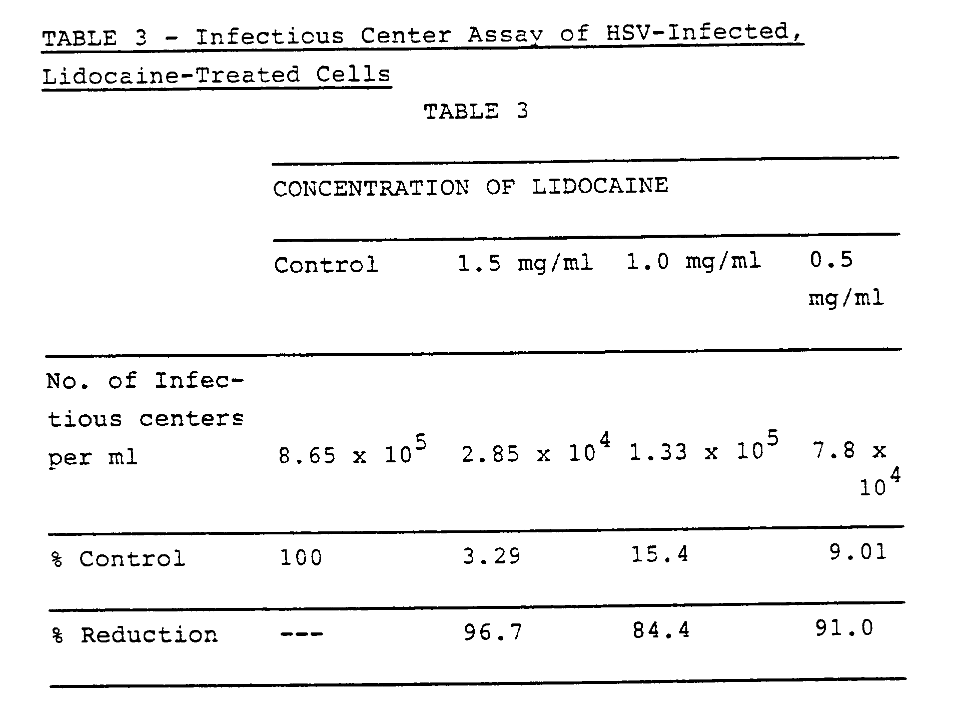

Vero cells were infected at an MOI Of 3. After a 1 hour adsorption period, lidocaine-containing maintenance media was added. After 18 hours incubation at 37°C, the cultures were washed three times in PBS, dispersed with trypsin-EDTA, and co-cultivated with uninfected Vero cells in HSV overlay medium for 72 hours. The overlay was then removed, the cells washed with PBS, stained with crystal violet, and the number of infectious centers counted.

### FIGURE 1 - Effect of Time of Treatment on the Replication Cycle

Vero cell cultures were infected with an MOI of 3 of HSV-1. After 1 hour of incubation at 37°C, maintenance media was added to all cultures, except the 1 hour post-treatment series to which either lidocaine or iododeoxyuridine-containing media was added. At 2, 4, 6, 8, 10, 12, 14, 16, and 18, hours, the remaining cultures were treated with the appropriate drug. All cultures, including six untreated (control) cultures, were harvested for intracellular virus at 18 hours post infection. The virus yield was determined by plaque assay, and the yields were plotted as the percent of virus produced at each post-infection treatment time as compared to the control yields. The results are given in Figure 1.

These results have adequately demonstrated that lidocaine hydrochloride and dexpanthenol are effective as antiviral agents in cell culture and act by inhibiting the replication herpes simplex virus in cell cultures.

Additional studies carried out indicate that lidocaine hydrochloride in combination with dexpanthenol in either topical or parenteral forms is effective in reducing the severity of developing herpes simplex type 1 lesions in hairless mouse model system when given at the tame of infection of the mice or when treatment is continued for one week after infection. It is also effective in reducing the severity of developing HSV-1 lesions in hairless mice when injected at the time of initial onset of the lesions. The results of these experiments are reported below.

### B. ANIMAL EXPERIMENTS

### EXPERIMENT ONE

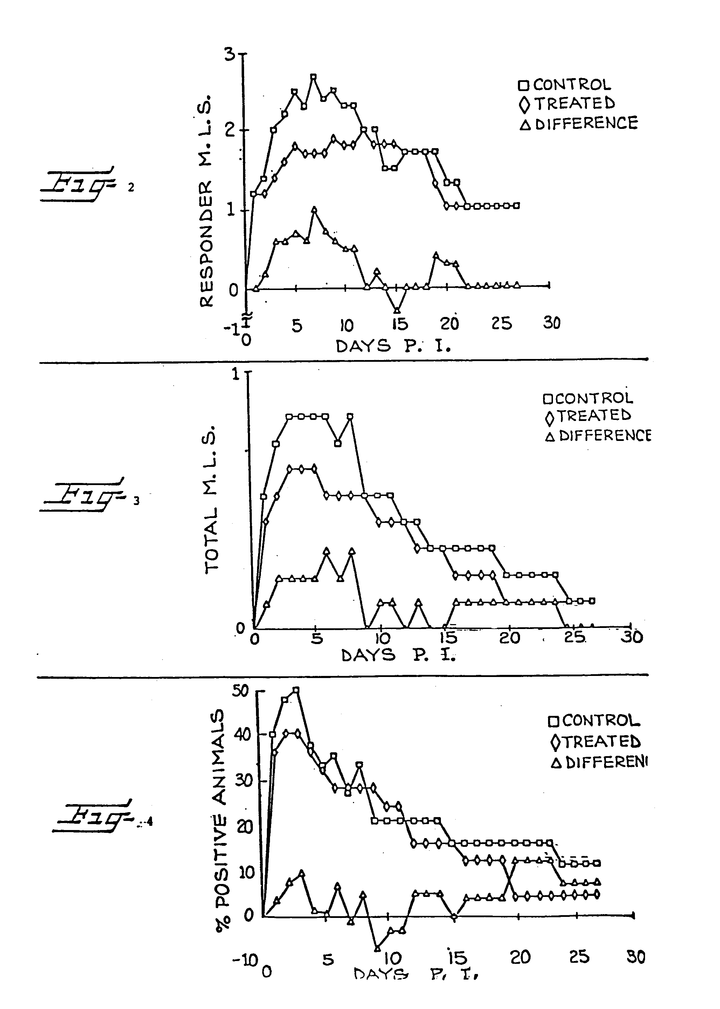

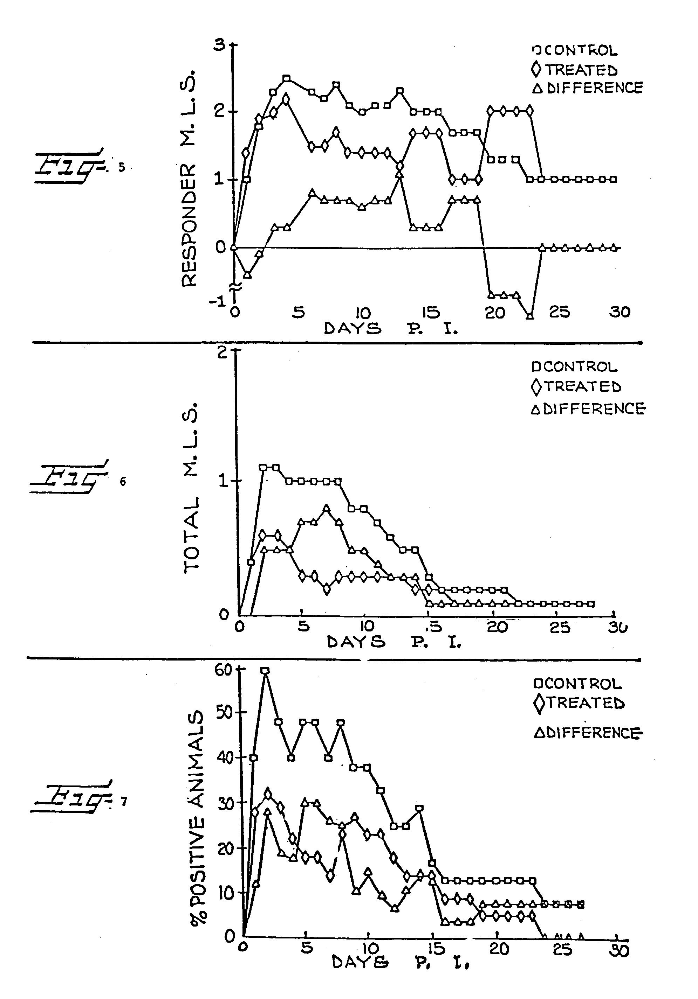

In this experiment, animals were scratch inoculated with HSV-1 and placebo (Group 1) or lidocaine hydrochloride-dexpanthenol (Group 2) ointment (lidocaine 40 mg/ml, para aminobenzoic acid 5%, panthenol 50 mg/ml in a base of wheat germ oil, cetyl alcohol, petrolatum, steryl alcohol, Tween 80, water, succinic acid, and ascorbic acid added as preservative and antioxidant) was applied ten minutes later and one additional time on the first day. The animals were then treated daily for seven days. Figure 2 shows the results of the responder mean lesion scores for the placebo and the treated animals. The responder mean lesion scores are defined as the cumulative lesion score of those animals which actually had lesions divided by the total number of animals with lesions. In this and every following figure, the control (placebo group) is indicated by squares and the treatment group is indicated by diamond shaped symbols. The arithmetical difference between the treatment and the control group is indicated by triangles. The results of this experiment indicate there is a significant difference (P < 0.005) between the control animals and the prophylactically treated animals. Lidocaine hydrochloride-dexpanthenol ointment applied daily after the time of scratch inoculation prevents the development of lesions as severe as those in the control group to a significant degree. This effect is seen for the first ten days of lesion development and healing. The scores even out after approximately ten days. Figure 3 compares the same groups of animals with respect to the total mean lesion scores. Total mean lesion divided by the total number of animals. This system of analysis takes into account those animals which did not develop lesions. In Figure 3, it is seen that there is a significant difference between the treated animals and the control group at approximately the same level as seen in Figure 2. The level of significance is P < 0.005. This indicates that the two methods of data evaluation do not produce a difference in the outcome. Figure 4 compares the total number of animals which developed lesions in the placebo and treatment groups and indicates that there was not a significant difference in the number of animals which developed lesions. These results indicate in this experiment that lidocaine hydrochloride-dexpanthenol ointment, when applied at the time of scratch inoculation with herpes simplex virus Type 1 in hairless mice, reduces the severity of developing lesions to a statistically significant degree.

### EXPERIMENT TWO

In this experiment, the animals were scratch innoculated with HSV-1 and cutaneous lesions were allowed to develop. The results are given in Figures 5 through 7. At the time of the first appearance of lesions, lidocaine hydrochloride-dexpanthenol solution was injected intramuscularly into the animals one time daily for one week. (Concentration of lidocaine hydrochloride per injection was 20 mg/ml; concentration of dexpanthenol was 30 mg/ml). Approximately 0.1 milliliter was injected. The purpose of this group was to see if systemic lidocaine hydrochloride in combination with dexpanthenol could be used to successfully treat cutaneous HSV-1 lesions in the infected hairless mice after onset of the lesions. A placebo injectable solution was used as a control.

Figure 5 indicates that there is a significant difference between the control and treated groups in the responder mean lesions score analysis (P < 0.001).

Figure 6 similarly indicates a difference in the total mean lesion score between the place and the control group.

Figure 7 indicates that the treatment group contains somewhat fewer animals that developed lesions than a control group. This is an insignificant finding since treatment was begun after lesions developed. These results indicate that injectable form lidocaine hydrochloride-dexpanthenol mixture is effective in reducing the severity of herpes simplex Type 1-induced lesions in hairless mice when given at the time the first lesions initially appear in the animals.

### NEW MICE TESTS

Through the techniques for the inoculation of the hairless mouse model system of HSV-1 given above, a series of experiments were conducted, designed to test different concentrations of lidocaine hydrochloride and dexpanthenol in the treatment of herpes simplex virus Type 1-induced skin lesions in the hairless mouse model system. The results of these experiments are given in Tables 4 and 5.

### RESULTS

Table 4 gives the mean lesion scores recorded on days 5, 10, 15, 20, and 25 after initial inoculation of herpes simplex virus Type 1 (HSV-1) in the hairless mouse model system for a wide range of combinations of concentrations of lidocaine and dexpanthenol. The results given in Table 4 indicate that for all concentration ranges of the combination of the two drugs, there is an effective reduction of the lesion size and severity at the days given compared to the untreated control animals. The only exception to this is with 0.5% lidocaine mixed with 3% dexpanthenol (Formulation A) in which the lesion score is approximately equivalent to that of the untreated controls (Formulation N), and 5% dexpanthenol alone (Formulation L) containing no lidocaine in which the lesion score is similar to that of the control. All other combinations of drugs showed effectivity in treating HSV-1 induced lesions as compared to the control solutions. It should be pointed out that 0.5% lidocaine is the lowest concentration of lidocaine used.

Table 5 gives the days to complete healing of the viral-induced lesion in the same groups of animals treated with the various concentrations of lidocaine hydrochloride and dexpanthenol. As seen in Table 5, it took less time for the lesions to reach complete healing in each group of animals treated with the various combinations of lidocaine and dexpanthenol than it did in the untreated controls or with dexpanthenol or lidocaine alone. Two exceptions to this were the lidocaine-dexpanthenol mixture at which lidocaine was in the concentration of 0.5% (Formulation A), which took 21.3 days to heal, and the 6% lidocaine hydrochloride and 0.5% dexpanthenol mixture (Formulation F) in which it took 23.5 days to heal. All other groups showed significant effect in reducing the healing time of HSV-1 induced lesions in the hairless mouse model system as compared to uninfected controls. The results of these experiments indicate that lidocaine-dexpanthenol mixtures throughout the range of concentrations given are effective in treating HSV-1 induced lesions in the hairless mouse model system except at very low concentrations.

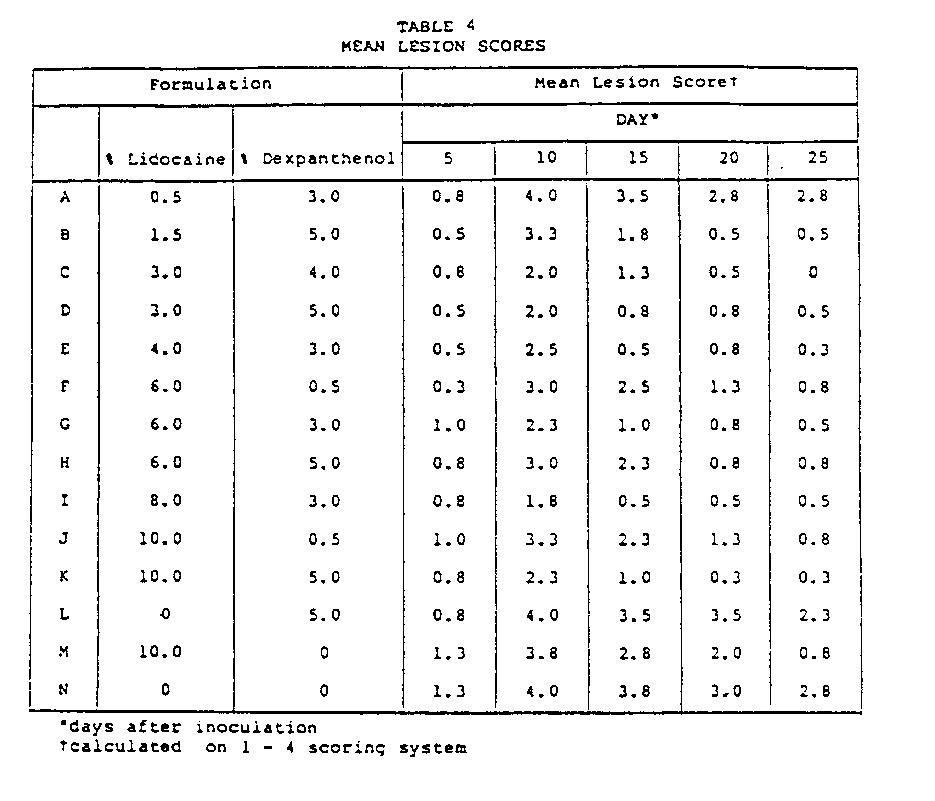

**TABLE 5**

| TIME TO COMPLETE HEALING | | | |
|---|---|---|---|
| Formulation | | | |
| | % Lidocaine | % Dexpanthenol | Days to Complete Heal (Mean) |
| A | 0.5 | 3.0 | 21.3 |
| B | 1.5 | 5.0 | 19.3 |
| C | 3.0 | 4.0 | 18.5 |
| D | 3.0 | 5.0 | 14.3 |
| E | 4.0 | 3.0 | 14.8 |
| F | 6.0 | 0.5 | 23.5 |
| G | 6.0 | 3.0 | 15.3 |
| H | 6.0 | 5.0 | 18.3 |
| I | 8.0 | 3.0 | 14.8 |
| J | 10.0 | 0.5 | 21.5 |
| K | 10.0 | 5.0 | 15.5 |
| L | 0 | 5.0 | >25 |
| M | 10.0 | 0 | 22.3 |
| N | 0 | 0 | >25 |

These results indicate in a scientifically determined manner that lidocaine hydrochloride and dexpanthenol, topically applied or injected, are able to successfully treat herpes simplex virus infections in mammals.

### HUMAN STUDIES WITH LIDOCAINE HYDROCHLORIDE

### C. HUMAN CASE STUDIES

### EXAMPLE 1

### MALE HERPES GENITALIS

This study comprised 14 cases, ranging in age from 16 to 50. All patients affected had had previous recurrent herpes of the genitals, with episodes recurring over periods of 1 to 6 months. The duration of genital episodes was between 10 to 14 days with intense burning before the appearance of blisters, followed by painful blisters before and after breaking, formation of scab, and remission. In this study, patients were seen before and 1 to 2 days after the appearance of blisters. They were advised to rub the affected area gently with a small amount of lidocaine hydrochloride-dexpanthenol in a specially prepared ointment base (lidocaine HCl 40 mg/ml, dexpanthenol 50 mg/ml in a base of wheat germ oil, cetyl alcohol, petrolatum, stearyl alcohol, Tween 80, water, succinic acid, and ascorbic acid as preservative and anti-oxidant) 3 times a day, in the morning, afternoon, and before going to bed.

### RESULTS

The pre-blister itching and pain associated with the blisters was markedly reduced or disappeared 15 to 20 minutes after the first application, In 6 out of 14 patients, itching and/or pain reappeared 2 to 3 hours after the first application, but symptoms were of a lesser intensity. After the second application, 8 to 10 hours later, itching, discomfort, and pain disappeared completely and did not reappear until the complete remission of the lesions. While the disappearance of the subject's symptoms--itching, discomfort, and pain--was expected due to the anesthetic effect of lidocaine, the unexpected and beneficial results obtained were the disappearance of blisters in 2 to 3 days, together with the disappearance of symptoms of local inflammation and discomfort. All cases treated showed a definite abortion or shortening of the 7 to 10 day itching, blister, scab cycle. They all cleared up after 2 to 3 days application of lidocaine hydrochloride ointment, regardless of the time when the treatment was instituted.

### EXAMPLE 2

### ANAL HERPES

One patient experiencing recurrent anal herpes was treated with 4% lidocaine hydrochloride, 5% dexpanthenol in a special ointment base listed in Example One Treatment was by rubbing the affected area gently with a small amount of the ointment 3 times a day in the morning, afternoon, and before going to bed. The patient showed definite improvement after 6 days with diminished pain. After 12 days, 75% improvement in pain and disappearance of eruptions. Previous anal herpetic episodes were lasting between 14 to 30 days with considerable pain and discomfort.

### EXAMPLE 3

### HERPES ZOSTER

Two patients with herpes zoster were treated with 4% lidocaine hydrochloride, 5% dexpanthenol in a special ointment base listed in Example One. Treatment was by rubbing the affected area gently with a small amount of the ointment 3 times a day, in the morning, afternoon, and before going to bed. One patient experienced increased pain during a 24-hour period, and the treatment was discontinued after one day. The other patient with herpes zoster of the head showed definite improvement after the first treatment and consistently improved thereafter with marked lessening of pain and eruptions.

### EXAMPLE 4

### HERPES OF THE HAND

One patient was treated with 4% lidocaine hydrochloride, 5% dexpanthenol in a special ointment base listed in Example One. Treatment was by rubbing the affected area gently with a small amount of the ointment 3 times a day, in the morning, afternoon, and before going to bed. Complete remission in 2 days.

### EXAMPLE 5

### HERPES OF THE FACE AND ORAL REGIONS (Herpes Labialis)

Five patients were treated with 4% lidocaine hydrochloride, 5% dexpanthenol in a special ointment base listed in Example One. Treatment was by rubbing the affected area gently with a small amount of the ointment 3 times a day, in the morning, afternoon, and before going to bed. Complete remission in 2 to 5 days and diminishing of pains and symptoms of the episode.

### EXAMPLE 6 - ADDITIONAL CASE REPORTS

The patient was a 25-year old male with genital herpes lesions. The site of the lesion was the shaft of the penis. There was stinging and a prickly feeling at the site of the lesion; mild itching, and no burning; with a history of recurrent (6 to 7 previous episodes) lesions. This patient was treated with a combination of lidocaine hydrochloride-dexpanthenol mixture in ointment form with 4% lidocaine hydrochloride, 5% dexpanthenol in a special ointment base listed in Example One and injectable form (lidocaine hydrochloride 20 mg/ml, dexpanthenol 30 mg/ml, niacinamide 20 mg/ml, riboflavin 1 mg/ml, thiamine 4 mg/ml, folic acid 100 ug/ml, citric acid 8 mg/ml, sodium citrate 12 mg/ml, benzyl alcohol 1.5% as preservative, and pH 4.5). Treatment was by rubbing the affected area gently with a small amount of the ointment 3 times a day, in the morning, afternoon, and before going to bed. The lesions were healed completely in 3 days with no pain and very little discomfort, and there was no new blister formation.

The patient was a 26-year old male. The site of the lesion was the dorsal area of the penis. The lesions were very mild; there was a sensation of slight burning. The patient has had herpes episodes for 3-4 years and several episodes of gonorrhea. This individual was treated with a combination of ointment with 4% lidocaine hydrochloride, 5% dexpanthenol in a special ointment base listed in Example One at the site of the lesions and injectable lidocaine (lidocaine hydrochloride 20 mg/ml, dexpanthenol 30 mg/ml, niacinamide 20 mg/ml, riboflavin 1 mg/ml, thiamine 4 mg/ml, folic acid 100 ug/ml, citric acid 8 mg/ml, sodium citrate 12 mg/ml, benzyl alcohol 1.5% as preservative, and pH 4.5). Treatment was by rubbing the affected area gently with a small amount of the ointment 3 times a day, in the morning, afternoon, and before going to bed. The lesions disappeared in 3 days. No new blisters were formed, and the pain disappeared.

The patient was a 34-year old female. The site of the lesion was the lip, lower right side; the lesion consisted of very small blisters. This patient was presented at 5 days into the episode had no prior episodes, and no history of venereal infections. This patient was treated with the ointment form with 4% lidocaine hydrochloride, 5% dexpanthenol in a special ointment base listed in Example One. Treatment was by rubbing the affected area gently with a small amount of the ointment 3 times a day, in the morning, afternoon, and before going to bed. The treatment was successful and complete remission occurred in 3 days.

The patient was a 23 year old female. The site of the lesions was just inside the vaginal orifice. There were burning and itching sensations. These were extremely tense lesions. The patient had no history of prior episodes, no history of venereal infections and was presented with lesions of a duration of about 5 days. The patient had tried several other presumed antiviral compounds with poor results. Upon treatment with lidocaine HCl-dexpanthenol ointment with 4% lidocaine hydrochloride, 5% dexpanthenol in a special ointment base listed in Example One, and injectable form (lidocaine hydrochloride 20 mg/ml, dexpanthenol 30 mg/ml, niacinamide 20 mg/ml, riboflavin 1 mg/ml, thiamine 4 mg/ml, folic acid 100 ug/ml, citric acid 8 mg/ml, sodium citrate 223 mg/ml, benzyl alcohol 2.5% as preservative, and pH 4.5), the burning, pain and lesions disappeared within 4 days. Treatment was by rubbing the affected area gently with a small amount of the ointment 3 times a day, in the morning, afternoon, and before going to bed.

The patient was a 55-year old female. The site of the lesions was on the left hand at the base of the third and fourth fingers; the lesions were red, swollen, broken blisters, oozing, and with deep laceration at the base of the index finger. There was a drawing pain in hand, itching, and burning, with pain in axillary region. The patient previously had vesicular lesions on the lips for 10 days, which disappeared. The lesions on the hand appeared following lip involvement. The lesions on the hand had been there 1 week prior to treatment. Patient had a temperature of 101° on the day prior to the visit. On the 3rd day after treatment was commenced with lidocaine hydrochloride-dexpanthenol ointment with 4% lidocaine hydrochloride, 5% dexpanthenol in a special ointment base listed in Example One, and injectable form (lidocaine hydrochloride 20 mg/ml, dexpanthenol 30 mg/ml, niacinamide 20 mg/ml, riboflavin 1 mg/ml, thiamine 4 mg/ml, folic acid 100 ug/ml, citric acid 8 mg/ml, sodium citrate 12 mg/ml, benzyl alcohol 1.5% as preservative, and pH 4.5), the pain and swelling had subsided. Treatment was by rubbing the affected area gently with a small amount of the ointment 3 times a day, in the morning, afternoon, and before going to bed. All lesions disappeared within 2 weeks.

The patient was a 45-year old female. The site of the lesions was on the introitus of the vagina and was irritated and painful. This was a chronic condition, and the patient had been uncomfortable for 5 to 6 months. Treatment was commenced with lidocaine hydrochloride-dexpanthenol ointment with 4% lidocaine hydrochloride, 5% dexpanthenol in a special ointment base listed in Example One and injectable form (lidocaine hydrochloride 20 mg/ml, dexpanthenol 30 mg/ml, niacinamide 20 mg/ml, riboflavin 1 mg/ml, thiamine 4 mg/ml, folic acid 100 ug/ml, citric acid 8 mg/ml, sodium citrate 22 mg/ml, benzyl alcohol 2.5% as preservative, and pH 4.5), and the lesions disappeared within 3 days after a commencement of treatment. Treatment was by rubbing the affected area gently with a small amount of the ointment 3 times a day, in the morning, afternoon, and before going to bed.

### NEW CASE STUDIES

A female patient with lesions on her upper lip was treated with lidocaine hydrochloride (2%) and dexpanthenol (3%) contained in a vehicle solution of sodium citrate (12 mg/ml), citric acid (8 mg/ml), and benzyl alcohol (0.5%) as a preservative. She applied the solution 4 times daily directly to the lesions by opening the lesion and applying the solution with a cotton-tipped applicator. By the third day of application, there were fewer blisters, all blisters were drying up, and discomfort was no worse. On the fourth day, the lesion was completely healed.

A male patient in his middle 30's with severe herpes on the shaft of the penis, whose normal and average duration of healing was 10 days, applied lidocaine hydrochloride (2%) and dexpanthenol (3%) contained in a vehicle solution of sodium citrate (12 mg/ml), citric acid (8 mg/ml), and benzyl alcohol (0.5%) as a preservative 5 times daily directly to the lesions. The solution was applied liberally directly onto the lesion and allowed to dry. He reported the herpes lesions better after one day of treatment. There were fewer blisters after day three, and they were drying up. There was less discomfort starting on day two. The lesions were completed healed by the fourth day.

A 42-year old male with herpes on the buttocks applied lidocaine hydrochloride (2%) and dexpanthenol (3%) contained in a vehicle solution of sodium citrate (12 mg/ml), citric acid (8 mg/ml), and benzyl alcohol (0.5%) as a preservative to an outbreak on the right upper buttock which was approximately 12 mm in diameter. The solution was applied directly 3 times a day with a cotton-tipped applicator. After four days, it had completely cleared.

Approximately one month later, he had an outbreak on the left buttock and on the right side of the penal shaft simultaneously. Both were large lesions. The buttock lesion was approximately 12.5 x 3.2 mm. He applied the medication four times a day until the lesions healed, which was in seven days.

The same individual had an outbreak at the base of the spine 10 days after the previous episode. This lesion was below the waistline and approximately 19 mm in diameter. He applied the solution immediately upon the outbreak of the lesions and the noticing of a red area and itching. He applied it 4 times daily for three days and did not develop further lesions in that area.

Two days later, he developed blisters in a separate area on the upper right buttock approximately 50 mm lower and away from the original lesion. He applied lidocaine hydrochloride (2%) and dexpanthenol (3%) contained in a vehicle solution of sodium citrate (12 mg/ml), citric acid (8 mg/ml), and benzyl alcohol (0.5%) three times a day, and these lesions were completely healed by the third day.

Approximately one month later, this patient noticed a prodromal symptom indicated by an itching under the skin in the lower spine above the buttocks. He applied the solution two times on the first day, and on the second day, he noticed a further redness and itching in a small area and began to apply the medication four times daily. Two days later, no redness or itching was noticed, and a full lesion did not appear.

A female, 35 years old, with episodic herpes virus infections on the external genitalia, had approximately six vaginal-labial sores. She applied lidocaine hydrochloride (2%) and dexpanthenol (3%) contained in a vehicle solution of sodium citrate (12 mg/ml), citric acid (8 mg/ml), and benzyl alcohol (8.5%) as a preservative directly to the sores three times on the first day and four times a day thereafter with a cotton-tipped applicator stick. The initial reaction was that there was a slight burning upon the application of the medication and that there was no improvement for four days. The fifth day, there was slight improvement, and the sores were less painful. On day six, there was much more improvement. The pain of the sores was minimal, and the sores were healing. On the seventh day, there was no pain, and the sores were 95% healed. On the eighth day, the sores were completely healed.

A male in his middle 30's had fever blisters on the face and chin. This individual applied lidocaine hydrochloride (2%) and dexpanthenol (3%) contained in a vehicle solution of sodium citrate (12 mg/ml), citric acid (8 mg/ml), and benzyl alcohol (0.5%) as a preservative four times daily with a cotton ball, directly to the affected area. He reported that by the third day the fever blisters were better, by the fourth day, there were fewer blisters, they were drying up, and all the lesions were completely healed after five days.

A 33-year old female, with external genital herpes applied lidocaine hydrochloride (2%) and dexpanthenol (3%) contained in a vehicle solution of sodium citrate (12 mg/ml), citric acid (8 mg/ml), and benzyl alcohol (0.5%) as a preservative four times daily with a cotton-tipped applicator. Her previous complaint was that she had a prodromal itching and tingling which led to a rash. No full blown blisters were available. Application of the solution completely healed the rash after five days, did not lead to the development of new blisters or rash, and eased the tingling and pain immediately.

A 24-year old female had external genital herpes lesions on her vulvar and external vaginal area. The lesions consisted of a red rash that was itching. No blisters formed. She reported a recurring history of two times a month for these types of lesions. She applied lidocaine hydrochloride (4%), dexpanthenol (3%) in a vehicle solution containing sodium citrate (12 mg/ml), citric acid (8 mg/ml), and benzyl alcohol (0.5%) as a preservative. She applied the solution liberally over the rash area 5 times daily, allowing it to dry completely. She reported that the herpes was better after one day of application, that it started to dry up immediately upon the application, that the discomfort was less starting one day after the application, and that the rash had completely disappeared by the seventh day of application of the solution.

A 31-year old female had an external genital herpes lesion. This lesion was a single blister in the vulvar area. She applied lidocaine hydrochloride (4%), dexpanthenol (3%) in a vehicle solution containing sodium citrate (12 mg/ml), citric acid (8 mg/ml), and benzyl alcohol (0.5%) as a preservative directly to the lesion 4 to 5 times daily with a cotton-tipped applicator by opening the blister and soaking the lesion area with the solution. She reported that the herpes "felt" better after the first day, that it was drying up after day one, that the discomfort was less, directly attributable to the application of the formula, and that the lesion was completely healed after the fourth day of application.

## Claims

1. The use of lidocaine or a pharmaceutical acceptable salt thereof for the preparation of a pharmaceutical composition for the inhibition of herpes viral infection in mammals.

2. The use of lidocaine according to claim 1, wherein the prepartion is for topical use of the affected area.

3. The use of lidocaine according to claim 1, wherein the preparation is for parenteral use in the form of an aqueous solution.

4. The use of lidocaine according to claims 1 to 3, wherein the preparation has a concentration of from 1.5 to 10 % w/v.

5. The use of lidocaine according to claims 1 to 4, wherein the preparation is a combination of the lidocaine or the lidocaine salt with panthenol, pantothenic acid or its salt.

6. The use of lidocaine according to claims 1 to 5, wherein the pantothenic acid is administered at a concentration of from 5 to 50 mg/ml.

## Patentansprüche

1. Verwendung von Lidocain oder einem pharmazeutisch annehmbaren Salz davon zur Herstellung einer pharmazeutischen Zusammensetzung zur Hemmung von Herpes-Virus-Infektionen bei Säugern.

2. Verwendung von Lidocain nach Anspruch 1, worin das Präparat für die äußere Anwendung des befallenen Gebiets ist.

3. Verwendung von Lidocain nach Anspruch 1, worin das Präparat für parenterale Anwendung in der Form einer wäßrigen Lösung ist.

4. Verwendung von Lidocain nach einem der Ansprüche 1 bis 3, worin das Präparat eine Konzentration von 1,5 bis 10% G/V hat.

5. Verwendung von Lidocain nach einem der Ansprüche 1 bis 4, worin das Präparat eine Kombination aus Lidocain oder dem Lidocainsalz mit Panthenol, Pantothensäure oder deren Salz ist.

6. Verwendung von Lidocain nach einem der Ansprüche 1 bis 5, worin die Pantothensäure mit einer Konzentration von 5 bis 50 mg/ml verabreicht wird.

## Revendications

1. L'utilisation de lidocaine ou d'un sel de celle-ci pharmaceutiquement acceptable pour la préparation d'une composition pharmaceutique pour l'inhibition de l'infection virale de l'herpès dans les mammifères.

2. L'utilisation de lidocaine selon la revendication 1, dans laquelle la préparation est faite pour l'usage topique de la région affectée.

3. L'utilisation de lidocaine selon la revendication 1, dans laquelle la préparation est faite pour l'usage parenteral sous forme d'une solution aqueuse.

4. L'utilisation de lidocaine selon l'une des revendications 1 à 3, dans laquelle la préparation a une concentration à partir de 1,5 à 10% p/v.

5. L'utilisation de lidocaine selon l'une des revendications 1 à 4, dans laquelle la préparation est une combinaison de la lidocaine ou du sel de la lidocaine avec le panthénol, l'acide pantothénique ou ses sels.

6. L'utilisation de lidocaine selon l'une des revendications 1 à 5, dans laquelle l'acide pantothénique est administrée à une concentration à partir de 5 à 50 mg/ml.
